# NOUVEAU FASCICULE DE BREVET EUROPEEN

(11) **EP 1 108 424 B2**
(45) Date de publication et mention de la décision concernant l'opposition: **04.09.2013**
(45) Mention de la délivrance du brevet: 01.06.2005
(21) Numéro de dépôt: 00403533.3
(22) Date de dépôt: 15.12.2000
(51) Int. Cl.: A61K 9/22, A61P 11/00

(54) **Comprimé matriciel permettant la libération prolongée de trimétazidine après administration par voie orale**
Trimetazidinhaltige Matrixtablette zur verlängerten Wirkstofffreisetzung nach oraler Gabe
Matrix tablet for sustained release of trimetazidine after oral administration

(30) Priorité: 17.12.1999 FR 9915960
(43) Date de publication de la demande: 20.06.2001
(73) Titulaire: Les Laboratoires Servier, 92284 Suresnes Cedex (FR)
(72) Inventeur: Huet de Barochez, Bruno, 45140 Ingre (FR); Dauphant, Claude, 45160 Olivet (FR); Wuthrich, Patrick, 45000 Orleans (FR)
(74) Mandataire: Dossmann, Gérard

(56) Documents cités:
- EP-A- 0 673 649
- EP-A2- 0 207 638
- FR-A- 2 490 963
- US-A- 5 334 392
- DATABASE WPI Week 8644 Derwent Publications Ltd., London, GB; AN 1986-289031 XP002145828 "Sustained release tablets contg. water-soluble medicine - using ultramicropowder of enteric polymer base as matrix material" & JP 61 212517 A ((NICM) NIPPON CHEMIPHAR CO), 20 septembre 1986 (1986-09-20) & CHEMICAL ABSTRACTS, vol. 106, no. 8, 23 février 1987 (1987-02-23) Columbus, Ohio, US; abstract no. 55929, Y YOSHIYUKI, K SEIJI, Y HIRANOJO, O HEIICHI: "Long-lasting tablet formulations"

## Description

La présente invention a pour objet un comprimé matriciel permettant la libération prolongée de dichlorhydrate de Trimétazidine à un acide pharmaceutiquement acceptable après administration par voie orale.

La Trimétazidine ou 1-(2,3,4-triméthoxybenzyl) pipérazine est une molécule qui, en préservant le métabolisme énergétique de la cellule exposée à l'hypoxie ou à l'ischémie, évite l'effondrement du taux intracellulaire de l'adénosinetriphosphate (ATP). Elle assure ainsi le fonctionnement des pompes ioniques et des flux transmembranaires sodium-potassium et maintient l'homéostasie cellulaire.

Le dichlorhydrate de Trimétazidine est actuellement utilisé en thérapeutique pour le traitement prophylactique de la crise d'angine de poitrine, lors des atteintes choriorétiniennnes ainsi que pour le traitement des vertiges d'origine vasculaire (vertiges de Ménière, acouphènes).

Le dichlorhydrate de Trimétazidine est jusqu'à présent administré par voie orale à des doses de 40 à 60 mg/jour, sous forme de comprimés dosés à 20 mg ou de solution buvable dosée à 20 mg/ml. Ces deux formes sont des formes à libération immédiate. Le brevet FR 2 490 963 décrit la forme comprimé à libération immédiate. Le dichlorhydrate de Trimétazidine est rapidement absorbé et éliminé par l'organisme, sa demi-vie plasmatique étant inférieure à 6 heures. Ceci impose de fractionner l'administration du principe actif sur 2 ou 3 prises par jour afin d'assurer des taux plasmatiques suffisants. La posologie la plus fréquemment requise lors des traitements est de trois comprimés par jour. Or, la multiplication des prises quotidiennes constitue un risque d'oubli aussi bien chez les sujets en activité que chez les sujets âgés déjà polymédiqués.

Du fait d'une absorption rapide, ainsi que de la demi-vie de 6 heures, ces formes à libération immédiate conduisent à des taux sanguins faibles au moment de la prise suivante. Or, l'on sait l'importance de maintenir une protection myocardique efficace tout au long du nycthémère et en particulier au petit matin où les conséquences de l'ischémie sont les plus dramatiques. La couverture journalière n'étant pas complète avec la forme à libération immédiate, la demanderesse a mis au point une forme à libération prolongée permettant de couvrir parfaitement les 24 heures en assurant un taux sanguin suffisant entre deux prises tout en conservant après chaque prise un pic plasmatique important de façon à maintenir l'efficacité de la Trimétazidine, en préservant le métabolisme énergétique de la cellule exposée à l'hypoxie ou à l'ischémie et en évitant l'abaissement du taux intracellulaire d'ATP.

Elle permet également d'éviter les effets vasodilatateurs périphériques en stabilisant les débits sanguins ou les effets tensionnels.

La nouvelle formulation selon l'invention permet donc de conserver les caractéristiques positives de la formulation décrite dans le brevet FR 2 490 963 tout en permettant une meilleure couverture journalière, ce qui se traduit par une meilleure compliance et une protection permanente.

La présente invention concerne plus particulièrement un comprimé matriciel permettant la libération prolongée de Trimétazidine après administration par voie orale constitué d'une dichlorhydrate de matrice hydrophile caractérisée en ce que la libération prolongée est contrôlée par l'utilisation d'un polymère dérivé de cellulose présent dans la matrice.

Ce comprimé matriciel, administrable préférentiellement deux fois par jour, permet d'obtenir une libération prolongée de principe actif tout en conservant à chaque prise un pic plasmatique important. En effet, il permet d'obtenir après chaque prise des taux plasmatiques chez l'homme, supérieurs à 70 µg/l et de maintenir avant la prise suivante un taux plasmatique supérieur ou égal à 40 µg/l, ce qui n'était pas le cas avec le comprimé décrit dans le brevet FR 2 490 963 lorsque celui-ci était administré 3 fois par jour.

Le résumé Chemical Abstract du brevet japonais JP61212517 décrit des comprimés à libération prolongée d'un principe actif tel que la trimétazidine HCl qui comprennent un polymère entérique comme constituant de base de la matrice polymérique ainsi qu'une huile pour permettre la libération controllée du principe actif.

Les dérivés de cellulose utilisés dans la matrice selon l'invention sont les éthers de cellulose choisi parmi l'hydroxypropylcellulose, l'hydroxyéthylcellulose, l'hydroxyméthylcellulose, et l'hydroxypropylméthylcellulose.

Le dérivé de cellulose est préférentiellement l'hydroxypropylméthylcellulose. Le pourcentage de polymère dérivé de cellulose est compris entre 25 et 50 % de la masse totale du comprimé.

Des hydroxypropylméthylcelluloses de viscosité comprises entre 100 cP et 100 000 cP peuvent être utilisées. La viscosité préférée est égale à 4 000 cP.

Divers excipients sont ajoutés à cette matrice hydrophile comme les liants, les diluants, les lubrifiants et les agents d'écoulement. Parmi les liants, on utilise préférentiellement la polyvidone. Le pourcentage de polyvidone est compris entre 3 et 12 % de la masse totale du comprimé. Parmi les diluants, on utilise préférentiellement l'hydrogénophosphate de calcium dihydraté qui permet une meilleure fluidité et une meilleure compressibilité que d'autres diluants comme le lactose monohydraté. Le pourcentage d'hydrogénophosphate de calcium est compris entre 25 et 75 % de la masse totale du comprimé.

Parmi les lubrifiants, on peut citer à titre non limitatif le stéarate de magnésium, l'acide stéarique, le behénate de glycérol ou le benzoate de sodium. Le lubrifiant préféré est le stéarate de magnésium. Enfin, à titre préférentiel, on utilise la silice colloïdale comme agent d'écoulement.

La Trimétazidine utilisée dans les comprimés matriciels selon l'invention se trouve sous la forme de dichlorhydrate.

Le pourcentage de dichlorhydrate de Trimétazidine est compris entre 15 et 30 % de la masse totale du comprimé, préférentiellement entre 15 et 18 %.

L'homme de l'Art admet généralement que la cinétique de libération de comprimés matriciels est dépendante de la nature et de la quantité du composant essentiel de la matrice, à savoir ici le dérivé de cellulose.

Or, de manière surprenante, il apparaît que la cinétique de libération du comprimé matriciel selon l'invention n'est influencée ni par la quantité ni par la qualité du dérivé de cellulose utilisé.

En effet, différentes formulations réalisées avec d'une part des hydroxypropylméthylcelluloses de viscosité différentes et d'autre part avec des quantités variables d'hydroxypropylméthylcellulose de même qualité ont montré des cinétiques de libération équivalentes. Ceci implique qu'il existe une synergie spécifique entre le dérivé cellulosique et la Trimétazidine.

La présente invention concerne également le procédé de préparation du comprimé matriciel. Celui-ci peut être préparé par granulation humide suivie d'une compression, par granulation sèche suivie d'une compression ou par compression directe. De manière préférentielle, le procédé de préparation est une granulation humide suivie d'une compression.

La granulation humide est effectuée par mélange de la Trimétazidine, de la polyvidone et du diluant puis mouillage de ce mélange. Cette première étape permet de créer autour du principe actif un environnement hydrophile favorable à sa bonne dissolution et également d'obtenir un dosage unitaire le plus régulier possible.

Dans une seconde étape, le granulé obtenu précédemment est mélangé avec le dérivé cellulosique. A ce mélange sont alors ajoutés le lubrifiant et l'agent d'écoulement. La troisième étape est la compression du mélange lubrifié obtenu précédemment.

Les comprimés ainsi formés sont alors, si on le souhaite, enrobés selon une technique classique d'enrobage.

Les exemples suivants illustrent l'invention mais ne la limitent en aucune façon. Les comprimés matriciels décrits dans les exemples ont été préparés de la manière suivante :
- ***Stade A*** : Mélange de la Trimétazidine, de la polyvidone et de l'hydrogénophosphate de calcium dihydraté, puis mouillage du mélange au moyen d'une quantité suffisante d'eau purifiée, granulation puis séchage des granulés.
- ***Stade B*** : Mélange des granulés obtenu au stade A avec de l'hydroxypropylméthylcellulose.
- ***Stade C*** : Lubrification du mélange obtenu au stade B avec du stéarate de magnésium et de la silice colloïdale.
- ***Stade D*** : Compression du mélange lubrifié obtenu au stade C sur machine à comprimé rotative de manière à obtenir des comprimés ayant une dureté mesurée par écrasement diamétral d'environ 40 à 160 N.

### EXEMPLE 1 : Formulations de différents comprimés matriciels de Trimétazidine diversement dosés

**Tableau 1 :**

| *Formules unitaires de 3 types de comprimés* | | | |
|---|---|---|---|
| **Composé** | **Quantité (mg)** | | |
| | **F₁** | **F₂** | **F₃** |
| Trimétazidine, dichlorhydrate | 60 | 30 | 35 |
| Hydroxypropylméthylcellulose | 112 | 74 | 74 |
| Polyvidone | 13,3 | 8,7 | 8,7 |
| Hydrogénophosphate de calcium dihydraté | 92 | 85,9 | 80,9 |
| Stéarate de magnésium | 2,2 | 1 | 1 |
| Silice colloïdale, anhydre | 0,5 | 0,4 | 0,4 |
| ***Masse totale du comprimé*** | ***280*** | ***200*** | ***200*** |

### EXEMPLE 2 :

L'exemple 2 montre que différentes quantités d'hydroxypropylméthylcellulose n'ont pas d'influence sur la cinétique de dissolution du comprimé.

**Tableau 2 :**

| *Formules unitaires* / *Quantités variables d'HPMC* | | |
|---|---|---|
| **Composé** | **Quantité (mg)** | |
| | **F₄** | **F₅** |
| Trimétazidine, dichlorhydrate | 35 | 35 |
| Hydroxypropylméthylcellulose | 54 | 94 |
| Polyvidone | 10,1 | 7,3 |
| Hydrogénophosphate de calcium dihydraté | 99,5 | 62,3 |
| Stéarate de magnésium | 1 | 1 |
| Silice colloïdale, anhydre | 0,4 | 0,4 |
| ***Masse totale du comprimé*** | ***200*** | ***200*** |

Le tableau 3 montre les pourcentages de produit libéré en fonction du temps pour les formulations F₄ et F₅.

**Tableau 3 :**

| *Cinétique de libération* | | |
|---|---|---|
| **Temps (h)** | **Pourcentage de produit libéré (%)** | |
| | **F₄** | **F₅** |
| 1 | 41 | 38 |
| 2 | 59 | 59 |
| 3 | 80 | 77 |
| 4 | 97 | 96 |

### EXEMPLE 3 :

L'exemple 3 montre que différentes qualités d'hydroxypropylméthylcellulose n'ont pas d'influence sur la cinétique de dissolution du comprimé.

**Tableau 4 :**

| *Formulations* / *Qualités variables d'HPMC* | | | |
|---|---|---|---|
| **Composé** | **Quantité (mg)** | | |
| | **F₃** | **F₆** | **F₇** |
| Trimétazidine, dichlorhydrate | 35 | 35 | 35 |
| Hydroxypropylméthylcellulose 4000 cP | 74 | - | - |
| Hydroxypropylméthylcellulose 100 cP | - | - | 74 |
| Hydroxypropylméthylcellulose 100 000 cP | - | 74 | - |
| Polyvidone | 8,7 | 8,7 | 8,7 |
| Hydrogénophosphate de calcium dihydraté | 80,9 | 80,9 | 80,9 |
| Stéarate de magnésium | 1 | 1 | 1 |
| Silice colloïdale, anhydre | 0,4 | 0,4 | 0,4 |

Le tableau 5 montre les pourcentages de produit libéré en fonction du temps pour les formulations F₃, F₆ et F₇.

**Tableau 5 :**

| *Cinétiques de libération* | | | |
|---|---|---|---|
| **Temps (h)** | **Pourcentage de produit libéré (%)** | | |
| | **F₃** | **F₆** | **F₇** |
| 1 | 43 | 41 | 40 |
| 2 | 62 | 59 | 60 |
| 3 | 86 | 83 | 83 |
| 4 | 105 | 102 | 100 |

### EXEMPLE 4 : Etude de la cinétique plasmatique

La cinétique plasmatique a été étudiée après administration chez 12 volontaires sains du comprimé matriciel de formulation F₃ décrit dans l'exemple 1.
L'administration a été réalisée pendant 4 jours à raison de deux comprimés par jour.
La cinétique plasmatique du comprimé de formule F₃ a été comparée à celle d'un comprimé à libération immédiate (LI) administré pendant 4 jours à raison de trois comprimés par jour.

La formulation unitaire du comprimé à libération immédiate (LI) est la suivante :

| | |
|---|---|
| Trimétazidine, dichlorhydrate | 20 mg |
| Amidon de maïs | 26 mg |
| Mannitol | 34 mg |
| Polyvidone | 4 mg |
| Stéarate de magnésium | 1 mg |
| Talc | 5 mg |

La concentration plasmatique moyenne est donnée dans la figure 1.

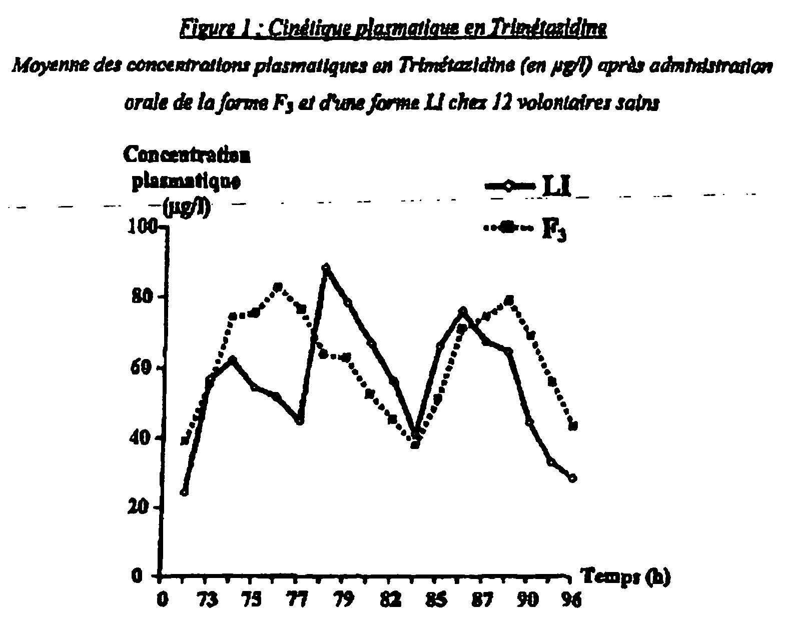

Cette courbe montre clairement que la forme F₃ permet d'obtenir une libération prolongée de Trimétazidine en conservant à chaque prise un pic plasmatique important.

En effet, le taux plasmatique observé après chaque prise est proche de 90 µg/l et peu différent de celui obtenu avec la forme LI. Ce taux est supérieur à 40 µg/l au bout de 24 heures, alors qu'avec la formulation à libération immédiate, celui-ci n'est que d'environ 25 µg/l.

## Revendications

1. Comprimé matriciel pour la libération prolongée de Trimétazidine sous forme de dichlorohydrate pharmaceutiquement acceptable, **caractérisé en ce que** la libération prolongée est contrôlée par l'utilisation d'un polymère dérivé de cellulose présent dans la matrice, choisi parmi l'hydroxypropylcellulose, l'hydroxyéthylcellulose, l'hydroxyméthylcellulose et l'hydroxypropylméthylcellulose, la trimétazidine sous forme de dichlorohydrate étant présente dans un pourcentage compris entre 15 et 30 % de la somme totale du comprimé et le pourcentage de dérivé de cellulose est compris entre 25 et 50 % de la masse totale du comprimé.

2. Comprimé matriciel selon la revendication 1, **caractérisé en ce que** le polymère dérivé de cellulose est l'hydroxypropylméthylcellulose.

3. Comprimé matriciel selon la revendication 1, **caractérisé en ce qu'**il contient également un liant, un diluant, un lubrifiant et un agent d'écoulement.

4. Comprimé matriciel selon la revendication 3, **caractérisé en ce que** le liant est la polyvidone.

5. Comprimé matriciel selon la revendication 4, **caractérisé en ce que** le pourcentage de polyvidone est compris entre 3 et 12 % de la masse totale du comprimé.

6. Comprimé matriciel selon l'une quelconque des revendications 3, 4 ou 5, **caractérisé en ce que** le diluant est l'hydrogénophosphate de calcium dihydraté.

7. Comprimé matriciel selon la revendication 6, **caractérisé en ce que** le pourcentage d'hydrogénophosphate de calcium dihydraté est compris entre 25 et 75 % de la masse totale du comprimé.

8. Comprimé matriciel selon l'une quelconque des revendications 3, 4, 5, 6 ou 7, **caractérisé en ce que** le lubrifiant est le stéarate de magnésium et l'agent d'écoulement la silice colloïdale anhydre.

9. Comprimé matriciel selon la revendication 1, **caractérisé en ce que** le pourcentage de dichlorohydrate de Trimétazidine est égal à 17,5 % de la masse totale du comprimé.

10. Comprimé matriciel selon l'une quelconque des revendications 1 à 9, **caractérisé en ce qu'**il contient 35 mg de dichlorohydrate de Trimétazidine, 74 mg d'hydroxypropylméthylcellulose, 8,7 mg de polyvidone, 80,9 mg d'hydrogénophosphate de calcium dihydraté, 1 mg de stéarate de magnésium et 0,4 mg de silice colloïdale anhydre.

11. Comprimé matriciel selon la revendication 10, **caractérisé en ce qu'**il est administré 2 fois par jour.

12. Comprimé matriciel selon l'une quelconque des revendications 1 à 11, utile pour le traitement prophylactique de l'angine de poitrine, lors des atteintes choriorétiennes ainsi que pour le traitement des vertiges d'origine vasculaire.

13. Procédé de préparation d'un comprimé matriciel selon l'une quelconque des revendications 1 à 10, **caractérisé en ce que** :
- l'on effectue une granulation humide par mélange de Trimétazidine, de polyvidone et du diluant puis mouillage de ce mélange,
- l'on mélange le granulé ainsi obtenu avec le dérivé cellulosique,
- l'on ajoute ensuite le lubrifiant et l'agent d'écoulement,
- puis l'on comprime le mélange précédent.

## Patentansprüche

1. Matrixtablette für die verlängerte Freisetzung von Trimetazidin in Form des pharmazeu-tisch annehmbaren Dihydrochloridsalzes, **dadurch gekennzeichnet, daß** die verlängerte Freisetzung durch die Verwendung eines in der Matrix vorhandenen von Cellulose abgeleiteten Polymeren ausgewählt aus Hydroxypropylcellulose, Hydroxyethylcellulose, Hydroxymethylcellulose und Hydroxypropylmethylcellulose gesteuert wird, wobei Trimetazidin in Form des Dihydrochlorids in einem Prozentsatz zwischen 15 und 30 % der Gesamtsumme der Tablette vorhanden ist und der Prozentsatz des Cellulosederivats zwischen 25 und 50 % der Gesamtmasse der Tablette beträgt.

2. Matrixtablette nach Anspruch 1, **dadurch gekennzeichnet, daß** das von Cellulose abgeleitete Polymere Hydroxypropylmethylcellulose ist.

3. Matrixtablette nach Anspruch 1, **dadurch gekennzeichnet, daß** sie zusätzlich ein Bindemittel, ein Verdünnungsmittel, ein Gleitmittel und ein Fließmittel enthält.

4. Matrixtablette nach Anspruch 3, **dadurch gekennzeichnet, daß** das Bindemittel Polyvidon ist.

5. Matrixtablette nach Anspruch 4, **dadurch gekennzeichnet, daß** der Prozentsatz des Polyvidons zwischen 3 und 12% der Gesamtmasse der Tablette beträgt.

6. Matrixtablette nach einem der Ansprüche 3, 4 oder 5, **dadurch gekennzeichnet, daß** das Verdünnungsmittel Calciumhydrogenphosphat-Dihydrat ist.

7. Matrixtablette nach Anspruch 6, **dadurch gekennzeichnet, daß** der Prozentsatz von Calciumhydrogenphosphat-Dihydrat zwischen 25 und 75% der Gesamtmasse der Tablette beträgt.

8. Matrixtablette nach einem der Ansprüche 3, 4, 5, 6 oder 7, **dadurch gekennzeichnet, daß** das Gleitmittel Magnesiumstearat und das Fließmittel ein wasserfreies kolloidales Siliciumdioxid ist.

9. Matrixtablette nach Anspruch 1, **dadurch gekennzeichnet, daß** der Prozentsatz des Trimetazidin-Dihydrochlorids 17,5 % der Gesamtmasse der Tablette beträgt.

10. Matrixtablette nach einem der Ansprüche 1 bis 9, **dadurch gekennzeichnet, daß** sie 35 mg Trimetazidin-Dihydrochlorid, 74 mg Hydroxypropylmethylcellulose, 8,7 mg Polyvidon, 80,9 mg Calciumhydrogenphosphat-Dihydrat, 1 mg Magnesiumstearat und 0,4 mg wasserfreies kolloidales Siliciumdioxid enthält.

11. Matrixtablette nach Anspruch 13, **dadurch gekennzeichnet, daß** sie 2-mal täglich verabreicht wird.

12. Matrixtablette nach einem der Ansprüche 1 bis 11, für die prophylaktische Behandlung der Angina pectoris, von chorioretinalen Vorfällen und für die Behandlung von Schwindelzuständen vaskulären Ursprungs.

13. Verfahren zur Herstellung einer Matrixtablette nach einem der Ansprüche 1 bis 10, **dadurch gekennzeichnet, daß** man:
- eine Feuchtgranulation durch Vermischen von Trimetazidin, Polyvidon und eines Ver-dünnungsmittels und Benetzen der Mischung bewirkt,
- die in dieser Weise erhaltene Granuliermischung mit dem Cellulosederivat vermischt,
- anschließend das Gleitmittel und das Fließmittel zusetzt,
- und dann die obige Mischung verpresst.

## Claims

1. Matrix tablet for the prolonged release of trimetazidine in pharmaceutically acceptable dihydrochloride form, **characterised in that** the prolonged release is controlled by the use of a cellulose derivative polymer present in the matrix, selected from hydroxypropylcellulose, hydroxyethylcellulose, hydroxymethylcellulose and hydroxypropyl methylcellulose, the trimetazidine in dihydrochloride form being present in a percentage of from 15 to 30 % of the total amount of the tablet, and the percentage of cellulose derivative is from 25 to 50 % of the total mass of the tablet.

2. Matrix tablet according to claim 1, **characterised in that** the cellulose derivative polymer is hydroxypropyl methylcellulose.

3. Matrix tablet according to claim 1, **characterised in that** it also comprises a binder, a diluent, a lubricant and a flow agent.

4. Matrix tablet according to claim 3, **characterised in that** the binder is polyvidone.

5. Matrix tablet according to claim 4, **characterised in that** the percentage of polyvidone is from 3 to 12 % of the total mass of the tablet.

6. Matrix tablet according to any one of claims 3, 4 or 5, **characterised in that** the diluent is calcium hydrogen phosphate dihydrate.

7. Matrix tablet according to claim 6, **characterised in that** the percentage of calcium hydrogen phosphate dihydrate is from 25 to 75 % of the total mass of the tablet.

8. Matrix tablet according to any one of claims 3, 4, 5, 6 or 7, **characterised in that** the lubricant is magnesium stearate and the flow agent is anhydrous colloidal silica.

9. Matrix tablet according to claim 1, **characterised in that** the percentage of trimetazidine dihydrochloride is 17.5 % of the total mass of the tablet.

10. Matrix tablet according to any one of claims 1 to 9, **characterised in that** it contains 35 mg of trimetazidine dihydrochloride, 74 mg of hydroxypropyl methylcellulose, 8.7 mg of polyvidone, 80.9 mg of calcium hydrogen phosphate dihydrate, 1 mg of magnesium stearate and 0.4 mg of anhydrous colloidal silica.

11. Matrix tablet according to claim 10, **characterised in that** it is administered twice per day.

12. Matrix tablet according to any one of claims 1 to 11 for use in the prophylactic treatment of angina pectoris, in chorioretinal attacks and in the treatment of vertigo of vascular origin.

13. Process for the preparation of a matrix tablet according to any one of claims 1 to 10, **characterised in that** :
- wet granulation is carried out by mixing trimetazidine, polyvidone and the diluent and then wetting that mixture,
- the granulate thus obtained is mixed with the cellulose derivative,
- the lubricant and the flow agent are then added,
- the previous mixture is then compressed.
